# EUROPEAN PATENT APPLICATION

(11) **EP 1 132 401 A1**
(43) Date of publication of application: **12.09.2001**
(21) Application number: 99947963.7
(22) Date of filing: 19.10.1999
(51) Int. Cl.: C07K 14/47, C07K 16/18, C12N 15/12

(54) **HUMAN PROTEINS STAM2a AND STAM2b AND cDNAS ENCODING THESE MOLECULES**

(30) Priority: 19.10.1998 JP 29646598
(71) Applicant: Japan Science and Technology Corporation, Kawaguchi-shi, Saitama 332-0012 (JP)
(72) Inventor: SUGAMURA, Kazuo, University of Tohoku, Med. Dept., Sendai-shi, Miyagi, 980-0872 (JP); ENDO, Kazuhiro, University of Tohoku, Med. Dept., Sendai-shi, Miyagi, 980-0872 (JP)
(74) Representative: Gardner, Rebecca
(86) International application number: JP9905756
(87) International publication number: WO0023468

(57) **Abstract**

This application provides human proteins STAM2a and STAM2b respectively containing the amino amid sequences of SEQ ID No. 1 and 2 which are novel signal transducers interacting with a cytokine signal transducer AMSH. This application also provides human gene encoding these proteins, their cDNAs respectively containing the nucleotide sequences of SEQ ID Nos. 3 and 4, and antibodies against STAM2a and STAM2b.

SEQUENCE LISTING
<110> Japan Science and Technology Corporation
<120> Human proteins STAM2a and STAM2b, and cDNAs encoding these molecules
<130> 99-F-053PCT/YS
<140> PCT/JP99/05756
<141> 1999-10-19
<150> JP No. 10-296465
<151> 1998-10-19
<160> 4
<210> 1
<211> 525
<212> PRT
<213> Homo sapiens
<400> 1
<210> 2
<211> 342
<212> PRT
<213> Homo sapiens

<400> 2

Met Pro Leu Phe Thr Ala Asn Pro Phe Glu Gln Asp Val Glu Lys Ala
1               5                   10                  15

Thr Asn Glu Tyr Asn Thr Thr Glu Asp Trp Ser Leu Ile Met Asp Ile
            20                  25                  30

Cys Asp Lys Val Gly Ser Thr Pro Asn Gly Ala Lys Asp Cys Leu Lys
        35                  40                  45

Ala Ile Met Lys Arg Val Asn His Lys Val Pro His Val Ala Leu Gln
    50                  55                  60

Ala Leu Thr Leu Leu Gly Ala Cys Val Ala Asn Cys Gly Lys Ile Phe
65                  70                  75                  80

His Leu Glu Val Cys Ser Arg Asp Phe Ala Thr Glu Val Arg Ala Val
                85                  90                  95

Ile Lys Asn Lys Ala His Pro Lys Val Cys Glu Lys Leu Lys Ser Leu
            100                 105                 110

Met Val Glu Trp Ser Glu Glu Phe Gln Lys Asp Pro Gln Phe Ser Leu
        115                 120                 125

Ile Ser Ala Thr Ile Lys Ser Met Lys Glu Glu Gly Ile Thr Phe Pro
    130                 135                 140

Pro Ala Gly Ser Gln Thr Val Ser Ala Ala Ala Lys Asn Gly Thr Ser
145                 150                 155                 160

Ser Asn Lys Asn Lys Glu Asp Glu Asp Ile Ala Lys Ala Ile Glu Leu
                165                 170                 175

Ser Leu Gln Glu Gln Lys Gln Gln His Thr Glu Thr Lys Ser Leu Tyr
            180                 185                 190

Pro Ser Ser Glu Ile Gln Leu Asn Asn Lys Val Ala Arg Lys Val Arg
        195                 200                 205

Ala Leu Tyr Asp Phe Glu Ala Val Glu Asp Asn Glu Leu Thr Phe Lys

    210                 215                 220

His Gly Glu Ile Ile Ile Val Leu Asp Asp Ser Asp Ala Asn Trp Trp

225                 230                 235                 240

Lys Gly Glu Asn His Arg Gly Ile Gly Leu Phe Pro Ser Asn Phe Val

                245                 250                 255

Thr Thr Asn Leu Asn Ile Glu Thr Glu Ala Ala Ala Val Asp Lys Leu

            260                 265                 270

Asn Val Ile Asp Asp Asp Val Glu Glu Ile Lys Lys Ser Glu Pro Glu

        275                 280                 285

Pro Val Tyr Ile Asp Glu Asp Lys Met Asp Arg Ala Leu Gln Val Leu

    290                 295                 300

Gln Ser Ile Asp Pro Thr Asp Ser Lys Pro Asp Ser Gln Asp Leu Leu

305                 310                 315                 320

Asp Leu Glu Asp Ile Cys Gln Gln Met Gly Pro Met Ile Asp Glu Lys

                325                 330                 335

Leu Glu Glu Ile Asp Arg

            340

<210> 3

<211> 1833

<212> DNA

<213> Homo sapiens

<220>

<221> CDS

<222> (111)..(1685)

<400> 3

TGAAGGCCGA AGCGGAGCGG TCTAGGGAGC CGCGGCCGCG GGTCACCCGG CGGGTAGCAG 60

TTGCTGAGTG TCAGCTAGAC AGCAGCGACT AGGGCTCGGG CGCCGGCGAG ATGCCTTTGT 120

TCACCGCCAA CCCCTTCGAG CAAGACGTGG AAAAAGCCAC GAATGAGTAC AACACTACAG 180

AAGATTGGAG TCTTATTATG GACATATGTG ACAAAGTTGG AAGTACTCCT AATGGAGCGA 240
AAGATTGCCT AAAAGCCATA ATGAAAAGGG TAAATCATAA GGTTCCACAT GTTGCTCTGC 300
AAGCACTAAC TCTTCTTGGG GCTTGTGTGG CAAACTGTGG AAAGATATTT CATTTAGAAG 360
TATGTTCCCG TGATTTTGCA ACAGAAGTAC GTGCTGTGAT TAAAAATAAG GCACATCCTA 420
AAGTATGTGA AAAACTGAAA TCTTTAATGG TGGAGTGGTC AGAAGAATTT CAGAAGGACC 480
CTCAGTTTAG TCTGATATCT GCAACTATTA AATCTATGAA AGAAGAAGGA ATTACTTTTC 540
CTCCAGCAGG TTCTCAGACT GTCTCAGCTG CTGCCAAGAA TGGTACGTCA TCGAACAAAA 600
ACAAAGAGGA TGAAGACATA GCTAAAGCTA TTGAATTATC GCTGCAAGAA CAGAAACAGC 660
AACACACAGA AACAAAATCC TTATATCCAT CTTCAGAAAT TCAGTTAAAT AATAAGGTTG 720
CACGGAAAGT GAGAGCTTTA TATGATTTTG AAGCTGTTGA GGACAATGAA CTCACCTTTA 780
AACATGGTGA AATAATTATT GTTTTGGATG ACAGTGATGC CAATTGGTGG AAAGGAGAAA 840
ATCACAGAGG AATAGGACTT TTCCCATCCA ATTTTGTAAC AACTAATTTA AACATAGAGA 900
CTGAGGCAGC GGCTGTGGAC AAATTGAATG TAATTGATGA TGATGTGGAG GAAATTAAGA 960
AATCAGAGCC TGAGCCTGTT TATATAGATG AGGATAAGAT GGATAGAGCC CTGCAGGTAC 1020
TTCAGAGTAT AGATCCAACA GATTCAAAAC CAGACTCCCA AGACCTTTTG GATTTAGAAG 1080
ATATCTGCCA ACAGATGGGT CCAATGATAG ATGAAAAACT TGAAGAAATT GATAGGAAGC 1140
ATTCAGAATT GTCTGAATTG AATGTTAAAG TCCTGGAAGC TCTGGAACTA TATAACAAAT 1200
TGGTGAATGA AGCACCAGTG TACTCAGTCT ATTCAAAGCT CCACCCTCCA GCACATTACC 1260
CACCTGCATC ATCTGGGGTT CCAATGCAGA CATATCCAGT TCAATCACAT GGTGGAAACT 1320
ATATGGGTCA GAGCATTCAC CAAGTAACTG TTGCCCAAAG CTATAGCCTA GGACCCGATC 1380
AAATTGGTCC ACTGAGATCT CTGCCTCCAA ATGTGAATTC CTCAGTGACA GCACAGCCTG 1440
CTCAAACTTC ATATTTAAGC ACTGGACAAG ACACTGTTTC CAATCCTACT TATATGAACC 1500
AGAACTCTAA CCTACAGTCA GCTACTGGTA CAACTGCTTA CAC
<210> 4
<211> 1368
<212> DNA
<213> Homo sapiens
<220>

<221> CDS

<222> (111)..(1136)

<400> 4

TGAAGGCCGA AGCGGAGCGG TCTAGGGAGC CGCGGCCGCG GGTCACCCGG CGGGTAGCAG 60

TTGCTGAGTG TCAGCTAGAC AGCAGCGACT AGGGCTCGGG CGCCGGCGAG ATGCCTTTGT 120

TCACCGCCAA CCCCTTCGAG CAAGACGTGG AAAAAGCCAC GAATGAGTAC AACACTACAG 180

AAGATTGGAG TCTTATTATG GACATATGTG ACAAAGTTGG AAGTACTCCT AATGGAGCGA 240

AAGATTGCCT AAAAGCCATA ATGAAAAGGG TAAATCATAA GGTTCCACAT GTTGCTCTGC 300

AAGCACTAAC TCTTCTTGGG GCTTGTGTGG CAAACTGTGG AAAGATATTT CATTTAGAAG 360

TATGTTCCCG TGATTTTGCA ACAGAAGTAC GTGCTGTGAT TAAAAATAAG GCACATCCTA 420

AAGTATGTGA AAAACTGAAA TCTTTAATGG TGGAGTGGTC AGAAGAATTT CAGAAGGACC 480

CTCAGTTTAG TCTGATATCT GCAACTATTA AATCTATGAA AGAAGAAGGA ATTACTTTTC 540

CTCCAGCAGG TTCTCAGACT GTCTCAGCTG CTGCCAAGAA TGGTACGTCA TCGAACAAAA 600

ACAAAGAGGA TGAAGACATA GCTAAAGCTA TTGAATTATC GCTGCAAGAA CAGAAACAGC 660

AACACACAGA AACAAAATCC TTATATCCAT CTTCAGAAAT TCAGTTAAAT AATAAGGTTG 720

CACGGAAAGT GAGAGCTTTA TATGATTTTG AAGCTGTTGA GGACAATGAA CTCACCTTTA 780

AACATGGTGA AATAATTATT GTTTTGGATG ACAGTGATGC CAATTGGTGG AAAGGAGAAA 840

ATCACAGAGG AATAGGACTT TTCCCATCCA ATTTTGTAAC AACTAATTTA AACATAGAGA 900

CTGAGGCAGC GGCTGTGGAC AAATTGAATG TAATTGATGA TGATGTGGAG GAAATTAAGA 960

AATCAGAGCC TGAGCCTGTT TATATAGATG AGGATAAGAT GGATAGAGCC CTGCAGGTAC 1020

TTCAGAGTAT AGATCCAACA GATTCAAAAC CAGACTCCCA AGACCTTTTG GATTTAGAAG 1080

ATATCTGCCA ACAGATGGGT CCAATGATAG ATGAAAAACT TGAAGAAATT GATAGGTGAG 1140

ATTTTTCGTG CAAGGAATAA AAAGTATAGT TTTGGGGGGT TCACTTGTGT TTTGGCAAAC 1200

AGTAAAGTAG GGAAAGTTTT TGTGGAACTG TACAATTCAT GGAAATTGTT TTTCAGTTTC 1260

CCAGGCTTTT AACAAATTTA TAGCAAGGTG GTGCTTTTTA TCTTGTTTTT GTTTTTCTAA 1320

AAAACAAGCT CTGACAAGTT TTATTAAATA AAAATTTTGC ATGGCTTA 1368

## Description

### Technical Field

The present invention relates to human proteins STAM2a and STAM2b, and the cDNAs encoding these molecules. More particularly, the present invention relates to novel signal transduction adapter molecules, STAM2a and STAM2b, human genes encoding these proteins, their cDNAs, and the antibodies against these proteins.

### Background Art

A variety of cells having different functions should collaborate with each other for expression of higher biological functions such as hematopoietic, immunological and nervous systems. Communication among the cells is essential for their collaborations. Cytokines are known to be the proteins responsible for intercellular communication, and include interleukin (IL)-1 to 18, colony stimulation factors (CSFs), interferons (IFNs) and several chemokines.

Signals are generated by binding of the cytokines to specific receptors on the cell membrane, and survival, proliferation, differentiation and functional expression of the cells are controlled by signal transduction. Accordingly, dysfunction of cytokine-receptor signal transduction pathways result in collapse of the immunological and hematopoietic systems to cause severe infectious diseases, cancers and autoimmune diseases.

For the reasons described above, it is quite important to elucidate the intracellular signal transduction pathways mediated by the cytokine/cytokine receptor system. This is important, in order to appreciate the basic phenomena such as proliferation and differentiation of the cells, pathogenesis, diagnosis and therapeutic intervention of various diseases at the molecular levels.

The inventors of the present invention have isolated the genes of "common y-chain" commonly included in a plurality of the cytokine receptors, and have made a great contribution in elucidating the structure and function of the cytokine receptors. Of particular elucidation is that the y-chain is an essential subunit for functional expression of IL-2, IL-4, IL-7 and IL-9, and abnormality in the y-chain causes impairment of early development of T-cells via dysfunction of IL-7 (Science, 262:1874-1877, 1993; Int. Immunol., 6:1451-1454, 1994; Science, 263:1453-1454, 1994; Eur. J. Immunol., 25:3001-3005, 1995).

Recently, the inventors of the present invention have identified "STAMs" as novel signal molecules concerning signal transduction in the proliferating cells by the cytokines, and found that these STAMs are present in the downstream of the IL-2/GM-CSF receptor and directly associate with JAK3/2. The inventors also found that STAMs play an important role in expression of c-myc and signal transduction for DNA synthesis (Immunity, 6:449-457, 1997).

While several important mechanisms of the intracellular signal transduction pathway by binding of the cytokines to the receptors have been made clear, additional novel molecules should be identified for elucidating the overall structure and function of the intracellular signal transduction pathway, because plural molecules are thought to be continuously and synergetically involved in the signal transduction pathway and to manifest final functional expression by constructing a so-called cascade.

### Disclosure of Invention

Accordingly, the object of the present invention is to provide adapter molecules similar to the STAMs that have been found by the inventors of the present invention.

Another object of the present invention is to provide a gene for these novel molecules and their cDNAs, and antibodies against these novel molecules.

The present invention for solving the above problems provides human proteins STAM2a and STAM2b having the amino acid sequences of SEQ ID Nos. 1 and 2, respectively.

The present invention also provides human genes encoding the human proteins STAM2a STAM2b described above; cDNAs of the human genes which have the nucleotide sequences of SEQ ID Nos. 3 and 4, respectively; and DNA fragments comprising partial sequences in the nucleotide sequences of SEQ ID Nos. 3 and 4, respectively.

The present invention further provides recombinant vectors containing the cDNAs or the DNA fragments described above, and antibodies against the human proteins STAM2a and STAM2b.

### Best Mode for Carrying Out the Invention

The procedures for obtaining the human proteins STAM2a and STAM2b of the present invention, and cDNAs for these proteins, as well as the procedures for identifying the functions of these proteins will be described first.

The human proteins STAM2a and STAM2b of the present invention are novel proteins obtained by the steps of isolating a sequence highly homologous to 5'-teminus of human STAM cDNA from the existing ESTs (Expression Sequence Tag), preparing an oligonucleotide probe based on the sequence, isolating respective cDNA clones by screening a human PHA stimulating leukocyte cDNA library, expressing these cDNA clones in a host cell, and isolating and purifying the novel proteins.

The human protein STAM2a has the sequence of SEQ ID No. 1 which consists of 525 amino acids and is encoded in the 111th to 1685th of SEQ ID No. 3 showing the nucleotide sequence of cDNA thereof. The protein has a molecular weight of 68 kDa and contains SH3 and ITAM regions as in STAM. STAM2a is 50.1% homologous to STAM. The human protein STAM2b has, on the other hand, the sequence of SEQ ID No. 2 consisting of 342 amino acids and is encoded in the 111th to 1136th of cDNA of SEQ ID No. 4. The protein STAMb2 may be conjectured to be a splicing variant of STAM2a in which the c-terminal including the ITEM region of STAM2a is deleted.

These proteins STAM2a and STAM2b are thought to have similar functions to STAM as adapter molecules in the signal transduction pathway involved in STAM. This suggestion may be confirmed by the following observations:
(1) STAM2a associates with Jak2 and Jak3 via the ITAM region;
(2) STAM2b does not associate with Jak2 and Jak3;
(3) A SH3 domain deleted variant of STAM2a suppresses DNA synthesis stimulated by IL-2 and GM-CSF; and
(4) Expression of c-myc gene stimulated by IL-2 and GM-CSF is accelerated by forced expression of STAM2a, however STAM2b does not have such acceleration activity.

The human proteins STAM2a and STAM2b can be obtained by using current method for isolating from human organs and cell lines. Preparing the peptides by chemical synthesis based on the amino acid sequences provided by the present invention, or a method for producing the proteins by the recombinant DNA technology using the cDNA fragments provided by the present invention. When the protein STAM2a or STAM2b is obtained by the recombinant DNA technology, for example, RNA is prepared by in-vitro transcription from the vector containing the cDNA of SEQ ID No. 3 or 4, and the protein may be expressed through in-vitro translation using the RNA as a template. Otherwise, by recombining an expression vector with the translation region of the cDNA by a known method in the art, the proteins encoded by cDNA may be obtained in a large scale in E. coli, bacillus subtilis, yeast, animal cells and plant cells.

When the proteins according to the present invention are produced by expression of DNA by in-vitro translation, the cDNA or its translation region is recombined into a vector containing a RNA polymerase promoter, and the recombinant cDNA is added in an in-vitro translation system such as a rabbit reticulocyte dyalizate or wheat germ extract containing a RNA polymerase for the promoter. Examples of the RNA polymerase promoter include T7, T3 and SP6. The vectors containing these polymerase promoter are, for example, pKA1, pCDM8, pT3/T7 18, pT7/3 19 and pBluescript II.

For large scale production of the protein encoded by cDNA in microorganisms such as E. coli, an expression vector is constructed by inserting cDNA according to the present invention or its translation region into an expression vector having an origin suitable for microorganism, a promoter, ribosome binding cite, cDNA cloning cite and terminator, followed by cultivation of the transformant obtained after transformation of the host cell with the expression vector. Protein molecules containing arbitrary regions may be obtained by permitting the cDNA to express by adding an initiating codon and a stop codon before and after an arbitrary translation region. Otherwise, only the desired protein portion may be obtained by allowing the protein to express as a fused protein with other proteins, followed by leaving the fused protein with an appropriate protease. Examples of the expression vector in E. coli include pUC, pBluescript II, pET and pGEX expression system vectors.

For producing the protein accord to the present invention in eukaryotic cells such animal cells, the cDNA or its translation region is inserted into an expression vector for the eukaryotic cells containing a promoter, splicing regions and poly(A) additional site to introduce the recombinant vector into the eukaryotic cells. Examples of the expression vectors include pKA1, pCDM8, pSVK3, pMSG, pSVL, pBK-CMV, pBK-RSV, EBV vector, pRS and pYES2. While mammal culture cells such as monkey kidney cells COS7 and Chinese hamster ovary cells CHO, budding yeast, dividing yeast silkworm cells and African clawed frog egg cells are usually used as the eukaryotic cells, any eukaryotic cells may be used so long as the cells can express the proteins according to the present invention. The methods known in the art such as an electroporation method, a calcium phosphate method, a liposome method and a DEAE dextran method may be used for introducing the expression vector into the eukaryotic cells.

Separation procedures known in the art may be combined for isolating and purifying the desired protein from the cultivation product after expressing the protein according to the present invention in microorganisms or eukaryotic cells. The separation and purification methods include, for example, treatment with urea or a denaturation agent, ultrasonic treatment, enzymatic digestion, salting out and solvent precipitation, dialysis, centrifugation, ultrafiltration, gel filtration, SDS-PAGE, isoelectric electrophoresis, ionexchange chromatography, hydrophobic chromatography, affinity chromatography and reversed phase chromatography.

Peptide fragments (more than five amino acid residues) containing any partial amino acid sequences of the amino acid sequence represented by the sequence numbers 1 or 2 are included in the proteins STAM2a and STAM2b according to the present invention. These peptide fragments may be also used for antigens for preparing antibodies. Fused proteins with other arbitrary proteins are also included in the proteins according to the present invention.

The gene according to the present invention is the human gene encoding the foregoing proteins, and can be isolated from the existing genome libraries using cDNA or its partial sequence as a probe.

cDNA according to the present invention can be obtained by screening the cDNA library derived from the human cells by colony or plaque hybridization method known in the art using the oligonucleotide synthesized based on the nucleotide sequence of cDNA of SEQ ID No. 3 or 4. Alternatively, cDNA according to the present invention may be also prepared from mRNA isolated from human cells by the RT-PCR method using a synthetic oligonucleotide that can hybridize to both terminus of the cDNA fragment as a primer.

Polymorphism due to individual difference is often observed in the human gene. Accordingly, cDNAs containing addition or deletion of one or plural nucleotides, and/or substitution with other nucleotides in SEQ ID No. 3 or 4 are also included in cDNA according to the present invention.

Likewise, proteins containing addition or deletion of one or plural amino acids, and/or substitution with other amino acids caused by alteration of cDNA may be included in the proteins according to the present invention, provided that the protein comprises protein activity of the protein having the amino acid sequence of SEQ ID No. 1 or 2.

The DNA fragment according to the present invention may include any cDNA fragments (10 bp or more) containing any partial nucleotide sequences of SEQ ID No. 3 or 4. DNA fragments comprising sense strand and antisense strand may be categorized into the DNA fragments according to the present invention. These DNA fragments can be used for a probe for gene diagnosis.

Antibodies against the proteins according to the present invention may be obtained as polyclonal or monoclonal antibodies by a conventional method using the protein itself or a partial peptide thereof as an antigen.

### Example

Applied on the PD-10 column was 5 mg of KLH (Keyhole Limpets Hemocyanin) after treating with a chemical cross-link agent (3-meleimidobenzoic acid N-hydroxy-succinimide) to obtain MBS linked KLH. A synthetic peptide of STAM2a (the amino acid number 17-29 represented by the sequence number 1) to the C-terminal of which cysteine was added was prepared, and a KLH linked STAM2a peptide was obtained by allowing the peptides to react with MBS linked KLH. A rabbit was immunized with this peptide as an antigen to obtain an antiserum. After precipitation of the antiserum with 40% saturation ammonium sulfate, the antiserum was purified by a protein A amity column to obtain an IgG antibody.

### Industrial Applicability

The present invention as hitherto described in detail provides novel adapter molecules related to the cytokine based signal transduction pathway and gene engineering materials. These molecules and gene engineering materials are useful for developing diagnostic and therapeutic methods of human diseases due to dysfunction of cytokine based signal transduction pathwaysuch as severe infectious diseases, cancers and autoimmune diseases.

## Claims

1. A human protein STAM2a having the amino acid sequence of SEQ ID No. 1.

2. A human protein STAM2b having the amino acid sequence of SEQ ID No. 2.

3. A human gene encoding the human protein STAM2a of claim 1.

4. cDNA of the human gene of claim 3, which is STAM2a cDNA having the nucleotide sequence of SEQ ID No. 3.

5. cDNA of the human gene of claim 3, which is STAM2b cDNA having the nucleotide sequence of SEQ ID No. 4.

6. A DNA fragment comprising a partial sequence in the nucleotide sequence of SEQ ID No. 3.

7. A DNA fragment comprising a partial sequence in the nucleotide sequence of SEQ ID No. 4.

8. A recombinant vector containing the STAM2a cDNA of claim 4 or the DNA fragment of claim 6.

9. A recombinant vector containing the STAM2b cDNA of claim 5 or the DNA fragment of claim 7.

10. An antibody against the human protein STAM2a of claim 1.

11. An antibody against the human protein STAM2b of claim 2.
